# EUROPEAN PATENT SPECIFICATION

(11) **EP 2 298 238 B1**
(45) Date of publication and mention of the grant of the patent: **11.11.2015**
(21) Application number: 09011991.8
(22) Date of filing: 21.09.2009
(51) Int. Cl.: A61F 2/04

(54) **Device for the treatment of lung associated conditions**
Vorrichtung zur Behandlung von mit der Lunge zusammenhängenden Leiden
Dispositif pour le traitement d'affections touchant aux poumons

(43) Date of publication of application: 23.03.2011
(73) Proprietor: Levin, Arnold Wolfovich, Barnaul 656045 (RU)
(72) Inventor: Levin, Arnold Wolfovich, 656045 Barnaul (RU); Razgulyaev, Evgeniy Nikolaevich, 656023 Barnaul (RU)
(74) Representative: Straus, Alexander

(56) References cited:
- WO-A-01/66190
- US-A1- 2003 127 090
- US-A1- 2004 055 606
- US-A1- 2004 060 563

## Description

The present invention relates to a multipurpose implantable device which may be used in the treatment of lung associates conditions. The device may be used for creating a hypoventilation in a lung area with the preservation of a drainage function from the bronchus. Due to its design the device may be used for blocking a bronchus independently of the bronchus' diameter, length and shape, and may be positioned by using essentially any bronchofiberscope.

### BACKGROUND OF THE INVENTION

The lung - also referred to as the pulmonary system - is the essential respiration organ in air-breathing animals. Lung associated conditions may thus easily turn out to severely challenge the well being of affected individuals and may even be life threatening. Of all lung associated conditions tuberculosis (TB) is one of the most common among humans. TB is caused by mycobacteria and often turns to a deadly infectious disease. The pathogens usually attack the lungs (as pulmonary TB) but may also affect the central nervous system, the lymphatic system, the circulatory system, the genitourinary system, the gastrointestinal system, bones, joints, and even the skin. According to the World Health Organization (WHO) about a third of the world population suffers from tuberculosis.

Recently, the number of severe and rapidly progressing lung tuberculosis forms leading to a lethal outcome has increased in Russia as well as in many other countries around the world. Among other reasons these forms are believed to be caused by the increased frequency of cases caused by drug-resistant strains of tuberculosis mycobacteria. The treatment of such cases turned out to be rather difficult and often even ineffective. In the meantime WHO gave out warnings of two new forms of tuberculosis, the so called MDR-and the XDR-TB. MDR-TB stands for "MultiDrug Resistant-Tubercolosis", a variant in which the pathogens have become resistant to many drugs and antibiotics. XDR-TB is even more dangerous: the "Extensively Drug-Resistant Tuberculosis" cannot be treated with any known drug or antibiotic. Hence, the role of other, non-medicated and slightly invasive methods in the treatment of tuberculosis such as an artificial pneumothorax has become more important in fighting this disease.

However, there exist some contraindications for such a treatment: acute progressive forms of lung tuberculosis (cheesy pneumonia and fibrous cavernous lung tuberculosis), bronchial tuberculosis, exudative pleurisy, pleural empyema, complete obliteration of the pleural cavity, impairment of blood clotting, and acute coronary pathology.

Other lung associated conditions include for example lung hemorrhage, an often fatal complication of different diseases, such as blood asphyxia with a development of hem-aspiration pneumonia.

Surgical treatment is usually recommended in most cases of drug resistant tuberculosis. Unfortunately most of these patients suffer extensive and acute forms of lung tuberculosis increasing the risk of complications and lethality during the post-operation period. Despite of considerable progresses in anesthesiology, reanimation procedures and intensive care therapies, post-operation lethality after thoracic surgery still varies from 9.1 % to 67.2 % and averages at 38.5 %. At the same time for most of these patients surgery has been considered to be the only opportunity saving their lives.

The most frequent and severe complications in thoracic surgery having a critical impact on the surgery outcome, the follow-up and the patient's life are bronchial fistula and a postoperative pleural empyema. The presence of bronchial fistula interferes with producing the vacuum necessary for straightening the lungs while the pleural empyema is an accumulation of pus - an exudate produced during inflammatory pyogenic bacterial infections in the pleural cavity. In its final stage pleural empyema leads to scarring of the pleural space and to lung entrapment.

Recently, various methods of treatment and devices have been developed in order to fight lung associated conditions and especially the most frequent and severe complications of destructive tuberculosis without primarily relying on drugs, antibiotics and especially on intensive thorax surgery.

The endobronchial valve therapy is known to be a minimally invasive treatment option for treating different forms of tuberculosis which are often complicated by pulmonary hemorrhage or other lung associated conditions such as emphysema. The treatment consists not only in prevention of asphyxia and hemoaspiration pneumonia but also in creating hypoventilation of the affected lung segment and preserving a drainage function of the blocked bronchus and the destructed cavity. To this end one (or more) endobronchial valve (a one-way valve) is installed in a bronchus. Such a valve enables air, sputum and other bronchial secretions to exit the lung during an abrupt expiration and a cough and at the same time blocks their return during inspiration. Thus a gradual condition of medicinal hypoventilation and pulmonary collapse is created in the obstructed part of the lung.

Endobronchial valves have been used since 1999 in the treatment of MDR-TB and various forms of such valves have been patented or disclosed in patent applications.

For example WO 2004/010845 describes a flow control device for a bronchial passageway which includes a valve member regulating fluid flow through the flow control device, a frame coupled to the valve member, and a membrane attached to the frame. A portion of the flow control device forms a seal with the interior wall of the bronchial passageway when the device is implanted in the bronchial passageway. The membrane of the device then forms a fluid pathway from the seal into the valve member in order to direct fluid flowing through the bronchial passageway into the valve member.

Russian patent 58898 dislcoses a reverse valve consisting of a hollow cylinder with radial foliated petals, which are conical and which have been truncated to a round opening that is fitted with a bridge. The device is made from a silastic composition but turned out to be not highly effective. Considering the variety of sizes (diameters), shapes and lengths of bronchi and bronchial fistulas it is not always possible to fix the valve in the bronchus that is needed to be blocked. After insertion this type of valve might even migrate and cause asphyxia.

Another device for treating lung tuberculosis is a valve in the form of a hollow cylinder, which has been presented to the public in Russian patent 2244517. The inlet and outlet aperture of the valve has an even round shape, the outer side is designed like a collapsible petal-like valve which may be locked by excessive external pressure. Thin foliated radial petals on two thirds of the exterior surface of the valve are aimed at fixing it in the desired location. Yet, also this device turned out not to be highly effective: the collapsible petal-like valve of the outlet aperture is longer than the hollow cylinder and protrudes from the cylinder's end into the bronchus. It therefore takes a floating position irritating the inner wall of the bronchus which eventually leads to exhausting coughs and inflammation of the bronchial mucosa.

Additionally, all of the devices of the prior art also suffer from the following drawback: in order to be successfully implanted, the valve's diameter must essentially exceed the blocked bronchus diameter by at least about 1.2 - 1.5 times. Since the valve's design is usually made with no consideration for the length and shape of the bronchus or bronchial fistula to be blocked, the physician must have access to a large number of valves of different diameters and must precisely pre-estimate the diameter of the bronchus before implanting a respective valve. In case the estimate was not precise enough, selecting the correct valve is often based on trial and error. Such a valve design also brings about the necessity for a huge selection of bronchofiberscopes since for every valve a different bronchofiberscope must be used as the inlet opening of the valve shall exceed the outer diameter of the bronchofiberscope by approximately 0.2 mm.

A device according to the preamble of claim 1 is known from the document US-A-2003/0127090.

At present, there is still a need for effective implantable devices to be used in the treatment of tuberculosis and other pulmonary associated conditions and their complications as the systems of the prior art proved to be imperfect and ineffective.

### SUMMARY OF THE INVENTION

The present invention is directed to a multipurpose device which may be implanted in essentially any body vessel irrespective of its diameter, length and shape to regulate body fluid flow.

In one embodiment the device, which for consistency shall be termed valvular bronchial blocker, is sized and configured to be positioned e.g. in a patient's lung such as e.g. a bronchus or bronchial fistula. The valvular bronchial blocker comprises a hollow cylinder having two openings, an inlet opening and an outlet opening, and a valve connected to the hollow cylinder such that a fluid flow is enabled through the valve only. According to an embodiment the valve is configured to allow fluid flow in a first direction only while substantially preventing fluid flow in a second direction. The hollow cylinder of the valvular bronchial blocker is formed like an accordion and may be extended by pressurizing its outer surface. The valve is connected to the hollow cylinder at a position, preferably to the inlet opening, while extending into the hollow cylinder. It opens at low pressures but closes quickly when airflow direction is reversed. It is designed to allow air and mucus to vent during exhalation and to prevent substances and fluids from entering the blocked bronchus. The one-way valve may be have any desired form, such as a poppet, ball, duckbill, heimlick, flap or leaflet valve.

In a preferred embodiment the valvular bronchial blocker may be configured to extend to a length two to ten times the length of a non-extended valvular bronchial blocker in its initial state, preferably three to nine times, three to seven times, three to five times, and most preferably to about four times the length of the non-extended valvular bronchial blocker.

In yet a further embodiment, the one-way valve is placed in the center of the hollow cylinder of the valvular bronchial blocker, where it is isolated and protected for consistent valve performance regardless of variations in airway anatomy or positioning.

In another embodiment the one-way valve does not extend beyond the outlet opening when the hollow cylinder is in an extended form.

In a further embodiment the one-way valve does not extend beyond the outlet opening when the hollow cylinder is in its initial state in a non-extended form.

In yet another embodiment of the present invention the inlet opening of valvular bronchial blocker has a bridge spanning the opening from one side to the opposing side. The bridge might serve as contact point for a bronchofiberscope or biopsy forceps. The bridge may be made of any material suitable for being implanted, such as a synthetic thread. It may also be made of a more rigid material so that the bridge may support the inlet opening. In a specific embodiment the bridge is made of titan.

In another embodiment of the present invention the valvular bronchial blocker comprises means for visualizing purposes, e.g. for making the valvular bronchial blocker visible when examining the patient, such as e.g. via X-raying, CT, MRT or ultrasonic waves. Such means may comprise incorporating e.g. a material or a mixture of materials into parts of the valvular bronchial blocker or applying it onto parts of the valvular bronchial blocker. The material may be located on the inside of the valvular bronchial blocker, the outside, or it is integrally formed with any of the structures of the valvular bronchial blocker such as the fluted tube, the bridge and/or the one-way valve. Preferably the visualizing means is located on the bridge or the bridge is formed of such means. Appropriate materials include all materials or mixtures thereof that are suitable for use in a patient and may comprise metals and alloys. In a preferred embodiment the material is a radio opaque material. In view of its antibacterial properties silver is a preferred material. In a further embodiment the material is titan.

In another embodiment of the present invention a metal framework isolates and protects the one-way valve to ensure consistent valve performance.

In a further embodiment parts or all of the valvular bronchial blocker, i.e. the hollow cylinder, the bridge and/or the valve are/is made of a biocompatible material, which may be porous or not and which is preferably essentially resilient, such as natural or synthetic rubber. Other suitable materials include but are not limited to molded silicone or urethane. In a special embodiment the valvular bronchial blocker is made of rubber stock IRP14-01 or rubber stock 52-336/4.

In yet another embodiment the valvular bronchial blocker is coated in medical grade silicone.

In another embodiment the hollow cylinder, the valve and/or the bridge are made of different materials. Preferably the valve is made of an isoprene rubber mix and the contour of the exterior surface of the valvular bronchial blocker is made of a porous rubber.

In a further embodiment hollow cylinder and the valve are integrally formed to yield the valvular bronchial blocker of the present invention.

### BRIEF DESCRIPTION OF THE DRAWINGS

Fig. 1 shows an endobronchial valve of the prior art.
Fig. 2 shows an implantable valvular bronchial blocker of the present invention before implantation in its non-extended form.
Fig. 3 shows an implantable valvular bronchial blocker of the present invention during or after implantation in an extended form.

### DETAILED DESCRIPTION OF THE INVENTION

From the technical viewpoint, the valvular bronchial blocker of the present invention provides improvements such as the possibility of being applied in essentially any bronchus or bronchial fistula irrespective of their diameter, length and form. Furthermore, because of the one-size fits all form of the valvular bronchial blocker it may be implanted by using essentially any bronchoscope or bronchofiberscope.

One embodiment of the present invention provides an implantable valvular bronchial blocker that allows fluid flow in a first direction and controls fluid flow in a second direction. As used herein, the term "fluid" shall designate gas, liquid, or a combination of a gas or gases and a liquid or liquids. In addition, the term "to control fluid flow" means that the flow is altered in some manner, i.e. a fluid may not flow unimpeded in a particular direction. The specific manner in which fluid flow is controlled in a particular direction depends on the construction of the valve of the present invention.

The valvular bronchial blocker of the present invention may, for example, completely block, substantially block, limit, meter or regulate fluid flow in a particular direction. As used herein, the term "one-way valve" is meant to highlight the aforesaid function of the valvular bronchial blocker and is not intended to limit the present invention to an endobronchial valve which completely blocks fluid flow in a specific direction. As used herein the term "essentially preventing fluid flow" shall designate that the fluid flow is controlled by either completely blocking, substantially blocking, limiting, metering or regulating fluid flow in the second direction.

For example, when positioned in a hollow structure in a patient's body, such as a bronchiole in one of the lungs, the valvular bronchial blocker is placed and oriented to allow fluid flow in the exhalation (first) direction and at the same time to prevent fluid flow in the inhalation (second) direction. The valvular bronchial blocker features a valve that opens during exhalation in order to deflate or decompress the isolated lung portion distal to the valvular bronchial blocker. Thereby the diseased tissue is maintained in sort of a decompressed state which prevents further hyper-expansion of the tissue.

The valvular bronchial blocker of the present invention may thus be used to prevent fluid from being drawn into one or more portions of a patient's lung.

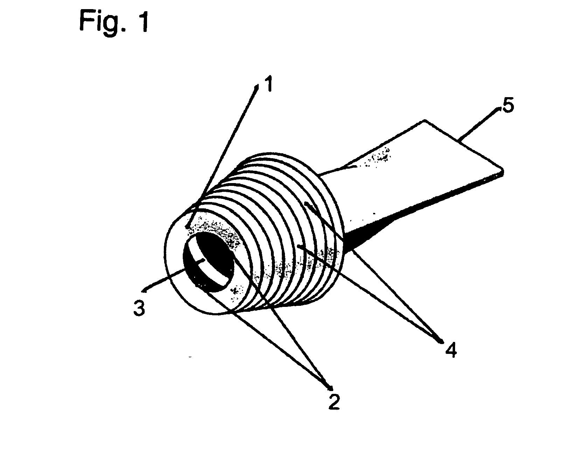

Referring to Fig. 1, which displays a known endobronchial valve, the general design of such a valve will be briefly highlighted. A typical endobronchial valve has the general appearance of a hollow cylinder 1. One side of the inlet mouth of the endobronchial valve defines the internal valve opening 2, having an even round shape. A bridge 3 usually holds open the internal valve opening 2 and serves as primary point of contact for a positioning device such as biopsy forceps and a bronchofiberscope. The opposite side is designed as a collapsible petal-like valve 5 which may be closed by excessive external pressure and the flexibility of the material it is made of. Thin foliated radial petals 4 on two thirds of the outer surface of the hollow cylinder 1 allow a firm fixation of the endobronchial valve in the bronchus.

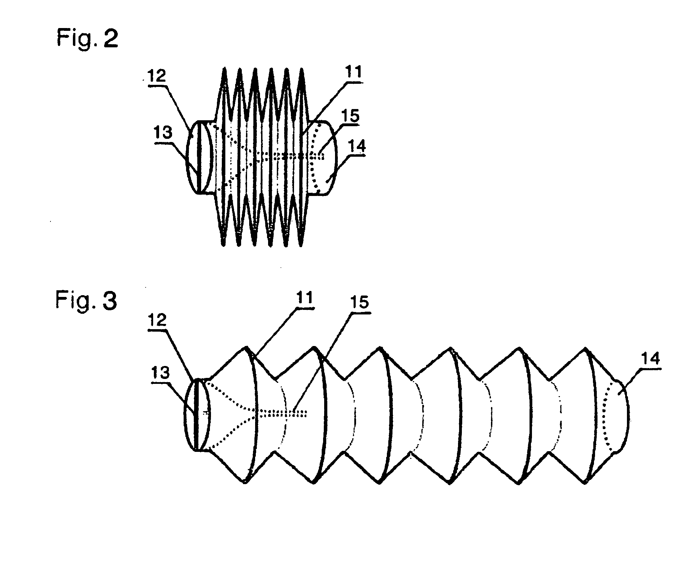

Referring now to Figs. 2 and 3, which show a preferred valvular bronchial blocker of the present invention in detail, the specific embodiment of the valvular bronchial blocker comprises a hollow cylinder in the form of a fluted tube 11 with radial foliated petals integrally formed on the cylinder. This structure is also referred to as accordion like. The fluted tube 11 exhibits on one end thereof an inlet opening 12 with a bridge 13 and on the opposite end thereof an outlet opening 14. The fluted tube design serves to adapt the diameter of the valvular bronchial blocker to that of the bronchus to be blocked so that a one size valvular bronchial blocker might be used for blocking essentially all bronchi, irrespective of the bronchus' diameter, length and shape. It further serves to anchor the device in the airway and to create an airtight seal against the bronchial wall to prevent fluid from leaking around the device. The fluted tube 11 provides a safe and reliable way for implanting the valve where it is necessary to avoid complications such as valve migration, inflammation and bronchial mucous membrane pressure sore. As the valvular bronchial blocker takes the shape of the blocked bronchus or bronchial fistula (detailed below), it is reliably fixed with no inflammation during treatment.

The inlet opening 12 is equipped with a collapsible petal-like one-way valve 15 which in this specific embodiment is totally located inside the fluted tube 11 and is thus protected and shielded against the outside. The valve 15 has two flaps connected to inlet opening 12 for forming an opening. The flaps extend towards the outlet opening 14 and the valve becomes narrower in that direction. According to the preferred embodiment the flaps end before reaching said outlet opening 14, thereby a floating position of the flaps within the bronchus is eliminated and an irritation that might lead to exhausting coughing and inflammation of the bronchial mucosa is prevented. The flaps are preferably made of an elastomeric material.

The valve 15 of the valvular bronchial blocker controls fluid flow by completely blocking such flow in a direction. As such, the valve 15 effectively functions as a one-way valve. Alternative embodiments of the invention utilize flow control elements that control fluid flow in a particular direction without completely blocking such a flow.

Implantation of the valvular bronchial blocker may be conducted by any suitable device known in the art for such purpose, such as a rigid bronchoscope as well as by essentially any bronchofiberscope and might be carried out under general or local anesthesia. Following bronchial tree examination the bronchoscope/ bronchofiberscope is removed and the device is set on its distal end while bridge 13 spanning the inlet opening 12 is held with biopsy forceps. The valvular bronchial blocker is then inserted in its non-extended initial state into the bronchus with the inlet opening 12 ahead. Under general anesthesia the device might be set through the cone of a rigid bronchoscope right to the desired position. In case of local anesthesia, the device is carried through the glottis to the trachea and the bronchus to be blocked during a deep breath. The valvular bronchial blocker is then held in the bronchus with the biopsy forceps and the bronchofiberscope is removed but from the device. The gradually decreasing lumen diameter of a bronchus will then apply a pressuring force on the fluted tube/accordion design of the valvular bronchial blocker, forcing the valvular bronchial blocker to gradually extend proportionally to the decreasing diameter of the lumen, thereby proportionally reducing its outer diameter. The valvular bronchial blocker may be extended in a preferred embodiment to about four times the length of the non-extended state of the valvular bronchial blocker.

Depending on the envisioned position the valvular bronchial blocker is simply moved down the bronchus until the final destination is reached. Forceps are opened and removed from the device under visual control. The valvular bronchial blocker is then in close and impervious connection with the bronchus in a sealing fashion such that fluid in the bronchial passageway must pass through the valvular bronchial blocker in order to travel past the location where the valvular bronchial blocker is located. It will be understood that a bronchus' rigidity is limited so that the pressuring force necessary for extending the valvular bronchial blocker will occur as soon as the valvular bronchial blocker is brought in tight contact with the inner wall of the bronchus by pushing it down the bronchus.

Due to the specific form of the valvular bronchial blocker the approximate size of the target lumen, i.e. the hollow structure in which the valvular bronchial blocker will be placed must not be specifically determined prior to installation.

The valvular bronchial blocker of the present invention can be used in the treatment of lung tuberculosis, pleural empyema and residual pleural cavities complicated by bronchopleural fistulas, various hemorrhage-complicated pulmonary diseases, heterogeneous lungs emphysema, pulmonary cysts, chronic obstructive pulmonary disease (COPD), and pneumothorax. Moreover, it can help to avoid many complications such as decubital ulcer of bronchial mucosa, valve migration, and valve-caused asphyxia.

Even though the present invention has been described with reference to lung or bronchial conditions only, it will be understood that the present device may likewise be applied to any other body vessel, the fluid flow through which shall be regulated.

The following examples illustrate the invention without limiting it thereto.

### Example 1

Patient, 31 years old, was delivered to the clinic 1 hour after injury (assault by strangers) complaining about severe headaches, nausea, vomiting, severe pain in the left half of the thorax and lower abdomen, weakness. From anamnesis it was found that two weeks before the injury the patient received treatment in the therapeutic department of one of the city hospitals on account of pneumonia of the left lung's lower lobe complicated by an abscess. At admission the patient's condition was severe. The skin was pale, body temperature was 38.2°C. Breath frequency was 24 per minute, BP was 100/60. The pulse was 104 per minute, the beats were of weak filling and tension. Level of consciousness could be described as moderate torpor, reduced critical abilities, partial disorientation in place and in time. Hemorrhages from the mouth and nose. Contused wounds of the frontal and left temporal areas. Based on the results of clinical, laboratory and instrumental examinations the diagnosis was made as follows: severe polytrauma, acute craniocerebral trauma, skull base fracture in the area of frontal and middle cranial fossae, severe brain contusion with contusion focuses located in the left frontal lobe. Epidural clot V3.4 in the right frontal lobe, contused wounds of the frontal and left temporal areas. Blunt abdomen trauma, parenchymal organ breaking, hemoperitonium, closed uncomplicated fracture Th_{XI}, Th_{XII}, and acute postpneumonic abscess of the X segment of the left lung.

The patient was operated under emergency proceedings. Laparotomy was performed under narcosis. During inspection of the abdominal cavity, a flat tender haematoma was found in the vicinity of the left kidney, extending to the colon root. The left kidney was isolated. A 1.0 x 0.5 cm and 1.0 x 1.0 cm tear was found at the kidney hilus on the anterior and posterior surfaces stretching through the hilus to the renal calices-pelvis system without penetration. The tears were stitched with an atraumatic needle. Haemostatic sponge was applied on the anterior surface of the kidnev. No other damages were found in the abdomen cavitv. Three drainages were set in the perirenal fat, one drainage in the abdomen, one in the pelvis minor. The operative wound was closed layer-wise tightly.

After the operation the patient was kept under artificial lung ventilation on account of inadequate spontaneous breathing. A degree II lungs hemorrhage suddenly occurred with 400 ml of dark blood excreted at once. For this reason, an emergency bronchofiberscopy was performed during which it was found that on the right BS₄, BS₅, BS₆, BS₇, BS₈, BS₉, BS₁₀ up to bronchi of the VI order were obstructed with dark blood clots and thick phlegm, on the left the lower lobe bronchus was filled with blood clots. Following sanitation the patency of the bronchi of all levels was restored. The source of hemorrhage was BS₁₀ on the left, the hemorrhage stopped. Considering a high possibility of hemorrhage recurrence and severity of the patient's condition and to prevent asphyxia from hemorrhage, the bronchus of the basal pyramid on the left was blocked with the device of the present invention.

During control X-ray examination, left segmental atelectasis of the basal pyramid was detected in the form of a triangular shade with an apex pointed to the lung root. The left cupula of the diaphragm was uplifted. At ultrasonic examination a 76.4 x 89.5 x 98.6 mm hypoechogenic tissue sector of a triangular shape and heterogeneous structure with the volume up to 353 cu mm was detected in the lower lobe of the left lung.

Following intra-bronchial blocking with the device, the patient was kept in massive antibacterial (claforan, ceftriaxone, amicasin), cryoplasm-antienzyme, and disintoxication (infusion) therapy. Drainages were removed from the abdominal cavity on the third day after the operation. The patient was provided with supplementary artificial lungs ventilation for seven days. In the reanimation unit and in the severe polytrauma unit, the patient was transfused 2,300 ml of polyglukin, 2,400 ml of reopolyglukin, 970 ml of stored matched erythrocyte mass and 900 ml of freshly frozen plasma.

Control bronchoscopy seven days after implantation indicated that all lobar and segmental bronchi remained patent. There was a valve at the left of the bronchus of the basal pyramid, there were traces of the 'old' blood, and little amount of mucoid sputum more proximal of the valve in the bronchus of the basal pyramid. There was no blood inflow into the bronchial tree.

As a result of the treatment, the state of the patient considerably improved: the "meningeal signs" disappeared, intoxication and respiratory embarrassment reduced, lung hemorrhage did not reappear.

However, the patient was urgently operated on 13 days after admission with the diagnosis of an early adhesive bowel obstruction. Videolaparospic adhesiotomy, nasogastric intubation of a small intestine, drainage of abdominal cavity were performed.

Postoperative recovery was smooth. Bronchofiberscopy was performed on seven days after the second surgery. Bronchial blocker was removed by biopsy forceps. Mucous membrane of the bronchus of the basal pyramid in the location of bronchia! blocker had flight edema and hyperemia. Roentgenogram and CT scans of chest organs in S10 projection showed an indication of a local pneumosclerosis of the left lung.

The patient was discharged from the clinic in satisfactory state and examined 6 months later. He was found in a condition of health and working.

Thus, in this case, application of a valvular bronchial blocker of the present invention helped to stop the lung hemorrhage of the patient with acute abscess of the left lung and a concurrent severe polytrauma, and led to the patient's recovery.

### Example 2

A 30-year-old patient was admitted to a hospital with complaints about cough with mucoid sputum totaling up to 50 ml per day, exertional dyspnea, weakness, and hyperhidrosis.

According to the anamnesis, infiltrative tuberculosis of superior lobe of the right lung in the phase of destruction and semination, MBT+ was detected 4 1/2 years earlier.

Initially, the patient received treatment in accordance with the DOTS-1 program. Then, during the following 3 years he was treated based on an individual program. However, roentgenograms and linear tomography scans indicated negative dynamics in a form of a destruction cavity enlargement accompanied by formation of big fibrotic caverns in superior lobe of the right lung. MBT was still secreted. The patient was offered surgical treatment several times; he refused it. MBT strains resistant to H, R and Pt were found in sputum samples with the help of inoculation methods.

Roentgenograms in frontal and right lateral projection and linear tomography scans of detected a large 6 x 8 cm cavern with a surrounding pericavital infiltration and nidal shadow in S6 of the right and S1-2 of the left lung. The following was diagnosed: fibrous-cavernous tuberculosis of superior lobe of the right lung in a stage of infiltration and semination to both lungs, MBT+, Multi-drug resistance (H, R, Pt).

In the hospital, the patient was treated with Fg 0.4; Z 2.0; S 1.0, as well as with the application of artificial pneumoperitoneum - 700 ml a week.

One month following admission the valvular bronchial blocking of the proximal bronchus of the right lung was carried out during bronchofiberscopy under local anesthesia using an embodiment of the present invention. The next day after valvular bronchial blocking the roentgenograms showed reduction of the size of destruction cavity, and occurrences of hypoventilation of superior lobe of the right lung.

General blood analysis two days after surgery showed: erythrocytes - 4.8 x 10¹²/1, hemoglobin - 150 g/1, leukocytes - 7.5 x 10⁹/1, ESR - 32 mm/hour. The patient was discharged for out-patient treatment in satisfactory state.

The patient was examined 9 months after valvular bronchial blocking. General blood analysis showed: erythrocytes - 4.6 x 10¹²/1, hemoglobin - 144g/1, leukocytes -5.4 x 10⁹/1, ESR - 10 mm/hour. MBT were not found in multiple samples of sputum analyzed with the help of luminescent and bacterioscopic methods. Sputum inoculation on MBT showed negative results three times. Chest fluorography showed that superior lobe remained in atelectasis, destruction cavity was not findable. Computer tomography scanning showed fibroatelectasis of superior lobe of the right lung, the absence of a destruction cavity therefore was confirmed.

Taking into account lack of destruction cavities, it was decided to remove the endobronchial valve. Bronchofiberscopy under local anesthesia showed that signs of diffuse simple I-stage endobronchitis remained.

The endobronchial valve was located in proximal bronchus. After the removal of the endobronchial valve bronchus orifice S 1, S2, S3 became patulous, there were no discharges of sputum. The mucous membrane in proximal bronchus was intumescenced, hyperemic, there was a granulation tissue area of 4 x 3 x 3 mm on the bo ttom wall, which was completely removed by biopsy forceps during bronchofiberscopy; histological conclusion - granulation tissue.

The application of the valvular bronchial blocker of the present invention in the course of the complex treatment of the patient with fibrous-cavernous tuberculosis of superior lobe of the right lung with MDR MBT permitted termination of bacterioexcretion, stabilization of tuberculous process and destruction cavity closing, which helped to avoid surgical treatment for this patient.

### Example 3

Patient, 20 years old, arrived at clinic with complaints about intensive pain in right part of chest, which became stronger during deep breathing and cough, as well as about pain during walking.

X-ray inspection of chest showed full collapse of the right lung, mediastinum shadow was not displaced. With the help of clinical and instrumental methods of examination, the illness was diagnosed as right total spontaneous pneumothorax.

According to urgent indications, drainage of the right pleural cavity was performed in the II intercostal space along middle-clavicular line. Nonetheless, X-ray inspection of the chest showed that full collapse of the right lung remained; also, intensive discharge of air on drainage during deep breathing and cough was observed. In view of the lack of tendency of the lung to smooth out, additional drainage of the right pleural cavity in the IV intercostal space along postaxillary line was performed the following day. Drainages were connected to active aspiration. Persistent discharge of air from right pleural cavity on both drainages remained. Computer tomography chest scan seven days after admission showed reduction of the right lung volume as a result of presence of free gas in pleural cavity. Superior lobe of the right lung was bullously changed by lots of cavities 2.6 - 3.3 cm in diameter. Subcutaneous emphysema of chest soft tissues was situated on the right side. The left side superior lobe was also bullously changed by cavities up to 3 cm in diameter predominately in I and II segment, a small air strip was detected on the left paracostally and paramesially.

With the help of clinical and instrumental examination methods, the illness was diagnosed as primary lung emphysema, multiple bullas of superior lobes of both lungs, complicated by total right pneumothorax and partial left pneumothorax of DNP stage.

In view of remaining right bronchopleural fistula, the patient underwent bronchofiberscopy under local anesthesia on the eighth day and an endobronchial valve of the present invention was installed in the proximal bronchus. Discharge of air via drainages was reduced, but it still remained. Valvular bronchial blocking of VI right segmental bronchus was performed. Discharge of air from pleural cavity stopped, the lung smoothed out on active aspiration.

Computed tomography scan of five days after the minor surgery showed that air appeared in left pleural cavity, superior lobe of the right lung was not smoothed out completely. There were air bullas on tops of both lungs. Also, little reduction of right lung volume was defined as the result of right subcutaneous emphysema of superior lobe.

X-ray chest inspection the following day showed that the left lung was pressed with air on the half of its volume; the right lung was smoothed out; right subcutaneous emphysema reduced.

In view of appearance of left pneumothorax, the patient underwent drainage of the left pleural cavity in the II intercostal space along middle-clavicular line on the same day. The drainages were removed from the right pleural cavity the following day.

Air discharge during deep breathing and cough continued on left pleural cavity drainage during 10 days. In consideration of existence of bullas in superior lobe of the left lung, preserving lung-pleural fistula and left lung collapse, valvular bronchial blocking of the right superior lobe bronchus was performed using a device of the present invention. Discharge of air from pleural cavity stopped, lung smoothed out on active aspiration.

Control X-ray chest inspection showed that lungs smoothed out without any focal and infiltrative changes, left lung transparence reduced in top parts. There was no liquid and no air in pleural cavity. Mediastinum shadow was not displaced. Computed tomography chest scan showed that left superior lobe and lingular segments were atelectasised. There were small bullas on both sides. There was no liquid and no air in the pleural cavity. The patient was discharged from the clinic in satisfactory state with amelioration.

These examples show that an application of the device of the present invention in a complex therapy of spontaneous pneumothorax provides an effective treatment, which is not accompanied by complications even if the valve is located in the bronchusal tree for a long time. The device of the present invention was tested on 21 patients with lung tuberculosis, on 10 patients with pleural empyema and residual pleural cavities with the bronchopleural fistulas, on 6 patients with lung hemorrhages, on 4 patients with pneumothoraxes, on 3 patients with lung cysts, and on 3 patients with heterogeneous lung emphysema.

All patients showed positive dynamics. When the device of the present invention was used, none of the patients exhibited any type of complication which might be related to the application of device.

## Claims

1. A device adapted for being implanted into a bronchus comprising:
a hollow cylinder having two openings; wherein the hollow cylinder is shaped and extendible like an accordion, and **characterised by**
a single valve in fluid connection with one of the openings, allowing fluid flow through the cylinder via the valve only,
and
wherein the device does not include a self-expanding scaffold located on the inner or outer wall of the hollow cylinder.

2. The implantable device according to claim 1, wherein the valve permits fluid flow in a first direction and essentially prevents fluid flow in a second direction opposed to the first direction when the device is located within a bronchial lumen.

3. The implantable device according to claim 1 or 2, wherein the valve is one of a poppet, ball, duckbill, heimlick, flap or leaflet valve.

4. The implantable device according to claim 3, wherein the valve comprises two flaps extending into the hollow cylinder.

5. The implantable device according to any of the preceding claims, wherein the diameter of the hollow cylinder varies as the device is being extended.

6. The implantable device according to any of the preceding claims, wherein the valve does not extend beyond any opening when the hollow cylinder is in an extended form.

7. The implantable device according to any of the preceding claims, wherein the valve does not extend beyond any opening when the hollow cylinder is in its initial shape in a non-extended form.

8. The implantable device according to any of the preceding claims, further comprising a bridge spanning an opening.

9. The implantable device according to any of the preceding claims, further comprising means for making the device visible when examining the patient, preferably wherein the means is a radio opaque material.

10. The implantable device according to any of the preceding claims, wherein at least the hollow cylinder is made of a porous rubber material.

11. The implantable device according to any of the preceding claims, wherein all parts of the implantable device are made of a porous rubber material.

12. The implantable device according to any of the preceding claims, wherein all parts are integrally formed.

## Patentansprüche

1. Zur Implantierung in Bronchien angepasste Einrichtung, welche umfasst:
einen Hohlzylinder mit zwei Öffnungen, worin der Hohlzylinder wie ein Akkordeon geformt und ausziehbar ist, **gekennzeichnet durch**
ein einziges Ventil, das mit einer der Öffnungen in Fluidverbindung steht und einen Fluidstromdurch den Zylinder nur über das Ventil erlaubt,
und worin die Einrichtung kein auf der Innenwand oder Außenwand des Hohlzylinders angeordnetes, selbst-expandierendes Gerüst beinhaltet.

2. Implantierbare Einrichtung nach Anspruch 1, worin das Ventil einen Fluidstrom in eine erste Richtung erlaubt und einen Fluidstrom in eine zweite zur ersten Richtung entgegengesetzte Richtung im Wesentlichen verhindert, wenn die Einrichtung in einem Bronchial-Lumen angeordnet ist.

3. Implantierbare Einrichtung nach Anspruch 1 oder 2, worin das Ventil ein Kolben-, Kugel-, Stoßschaufellader-, Heimlick-, Klappen- oder ein Faltblatt-Ventil ist.

4. Implantierbare Einrichtung nach Anspruch 3, worin das Ventil zwei Klappen umfasst, die sich in den Hohlzylinder erstrecken.

5. Implantierbare Einrichtung nach einem der vorstehenden Ansprüche, worin der Durchmesser des Hohlzylinders sich ändert, wenn die Einrichtung auseinandergezogen wird.

6. Implantierbare Einrichtung nach einem der vorstehenden Ansprüche, worin sich das Ventil nicht über eine Öffnung erstreckt, wenn der Hohlzylinder in einer auseinandergezogenen Form vorliegt.

7. Implantierbare Einrichtung nach einem der vorstehenden Ansprüche, worin das Ventil sich nicht über eine Öffnung erstreckt, wenn der Hohlzylinder in seiner Ursprungsform in einer nicht-auseinandergezogenen Form vorliegt.

8. Implantierbare Einrichtung nach einem der vorstehenden Ansprüche, welche weiter eine eine Öffnung überspannende Brücke umfasst.

9. Implantierbare Einrichtung nach einem der vorstehenden Ansprüche, welche weiter Mittel zur Sichtbarmachung der Einrichtung bei einer Untersuchung des Patienten umfasst, vorzugsweise worin die Mittel röntgendichtes Material ist.

10. Implantierbare Einrichtung nach einem der vorstehenden Ansprüche, worin mindestens der Hohlzylinder aus einem porösem Gummimaterial besteht.

11. Implantierbare Einrichtung nach einem der vorstehenden Ansprüche, worin alle Teile der implantierbaren Einrichtung aus einem porösen Gummi-Material bestehen.

12. Implantierbare Einrichtung nach einem der vorstehenden Ansprüche, worin alle Teile einstückig ausgebildet sind.

## Revendications

1. Dispositif adapté pour être implanté dans une bronche, comprenant :
un cylindre creux possédant deux ouvertures dans lequel le cylindre creux est profilé et extensible comme un accordéon et **caractérisé par**
une valve unique en connexion de fluide avec une des ouvertures, permettant un écoulement de fluide à travers le cylindre par l'intermédiaire de la valve uniquement,
et où le dispositif n'inclut pas de structure auto-expansible située sur la paroi interne ou externe du cylindre creux.

2. Dispositif implantable selon la revendication 1, dans lequel la valve permet un écoulement de fluide dans une première direction et empêche essentiellement un écoulement de fluide dans une deuxième direction opposée à la première direction lorsque le dispositif est situé au sein d'une lumière bronchique.

3. Dispositif implantable selon la revendication 1 ou 2, dans lequel la valve est une parmi une valve à champignon, à boule, à bec de canard, de heimlich, à clapet ou à feuillets.

4. Dispositif implantable selon la revendication 3, dans lequel la valve comprend deux clapets s'étendant dans le cylindre creux.

5. Dispositif implantable selon l'une quelconque des revendications précédentes, dans lequel le diamètre du cylindre creux varie à mesure que le dispositif est en cours d'extension.

6. Dispositif implantable selon l'une quelconque des revendications précédentes, dans lequel la valve ne s'étend pas au-delà d'une quelconque ouverture lorsque le cylindre creux est sous une forme étendue.

7. Dispositif implantable selon l'une quelconque des revendications précédentes, dans lequel la valve ne s'étend pas au-delà d'une quelconque ouverture lorsque le cylindre creux est dans sa forme initiale sous une forme non étendue.

8. Dispositif implantable selon l'une quelconque des revendications précédentes, comprenant en outre un pont couvrant une ouverture.

9. Dispositif implantable selon l'une quelconque des revendications précédentes, comprenant en outre un moyen pour rendre le dispositif visible lors de l'examen du patient, de préférence dans lequel le moyen est un matériau radio-opaque.

10. Dispositif implantable selon l'une quelconque des revendications précédentes, dans lequel au moins le cylindre creux est constitué d'un matériau de caoutchouc poreux.

11. Dispositif implantable selon l'une quelconque des revendications précédentes, dans lequel toutes les parties du dispositif implantable sont constituées d'un matériau de caoutchouc poreux.

12. Dispositif implantable selon l'une quelconque des revendications précédentes, dans lequel toutes les parties sont formées en une seule pièce.
